# EUROPEAN PATENT SPECIFICATION

(11) **EP 4 297 805 B1**
(45) Date of publication and mention of the grant of the patent: **25.06.2025**
(21) Application number: 22707069.5
(22) Date of filing: 16.02.2022
(51) Int. Cl.: A61L 15/28, A61L 15/42, A61L 15/60, A61L 27/20, A61L 27/56, A61L 27/58, A61L 31/04, A61L 31/10, A61L 31/14

(54) **IMPLANTS WITH SWELLABLE NANOCELLULOSE**
IMPLANTATE MIT QUELLFÄHIGER NANOCELLULOSE
IMPLANTS DOTÉS DE NANOCELLULOSE GONFLABLE

(30) Priority: 24.02.2021 EP 21158977
(43) Date of publication of application: 03.01.2024
(73) Proprietor: BIOTRONIK AG, 8180 Bülach (CH)
(72) Inventor: RZANY, Alexander, 90449 Nuernberg (DE); ANDREE, Verena, 91052 Erlangen (DE); HENSEL, Bernhard, 91052 Erlangen (DE)
(74) Representative: Biotronik Corporate Services SE
(86) International application number: PCT/EP2022/053789
(87) International publication number: WO 2022/179909

(56) References cited:
- EP-A1- 3 459 566
- EP-A1- 3 459 566
- WO-A1-2016/083351
- WO-A1-2016/083351
- CN-A- 108 822 335
- CN-A- 108 822 335

## Description

The present invention relates to a method for producing a swellable body comprising bacterial cellulose as well as to a swellable body produced by such a method as well as to a medical implant comprising a swellable body according to the present invention.

For a variety of minimally invasive implant applications, an exceptionally swellable biomaterial is advantageous, or such applications are made possible in the first place by such a material. Examples include sealing rings on implants such as TAVI prostheses to prevent paravalvular leaks, stent systems to stabilize aneurysms, closure systems for the atrial auricle in the left atrium, and closure systems of holes or puncture sites.

Swellable biological materials based on tissues of animal origin are known in the state of the art. Particularly, WO 2016/083351 Al describes a swellable body made from bacterial cellulose.

Document EP 3 459 566 A1 discloses a method for producing a swellable (highly porous) body comprising bacterial cellulose, the method comprising the steps of : providing a body made of bacterial cellulose, cleaning the body with NaOH and then freeze drying said body.

Based on the above, the problem to be solved by the present invention is to provide a body comprising bacterial cellulose that is improved regarding its swelling capability.

This problem is solved by a method according to claim 1, a swellable body according to claims 10 and 11, and a medical implant according to claim 12, the implant comprising a swellable body according to the present invention. Embodiments of these aspects of the present invention are stated in the corresponding sub claims and are described below.

According to claim 1, a method for producing a swellable body comprising bacterial cellulose is disclosed, wherein the method comprises the steps of:
(i) providing a body made of bacterial cellulose,
(ii) cleaning the body using at least one liquid medium,
(iii) freezing the body at atmospheric pressure for at least six hours and
(iv) freeze drying the body, and
(v) mechanically pressing the entire body (10) or parts thereof after freeze drying of the body (10).

Particularly, freeze drying is also known as lyophilization or cryodesiccation and corresponds to a low temperature dehydration process that comprises freezing the body and lowering pressure of an atmosphere surrounding the body and removing the ice by sublimation to dry the body. The body formed with the method according to the present invention is herein also denoted as a material.

Particularly, the swellable body produced using the method according to the present invention can be used in implants that incorporate an integrated seal for leakage, or are capable of filling cavities or seal openings. For this purpose, particularly, a special processing of bacterial cellulose, particularly nanocellulose, is used, which surprisingly leads to an enormous and unique swelling capacity.

Advantageously, the method for producing the swellable body for the production of a pressed, dry bacterial nanocellulose prevents the formation of intermolecular hydrogen bonds between the cellulose fibrils during drying or during pressing, the so-called cornification. Particularly, this results in an extremely high swelling capacity of more than 2000% by volume when coming in contact with an aqueous medium i.e. it becomes more than twenty times thicker, which is unique for pure bacterial cellulose. Advantageously, the method allows the production of virtually any three-dimensional molded part for use in a wide variety of implant classes. Furthermore, bacterial cellulose is a very blood- and biocompatible, non-degradable biomaterial, which is already approved in medical applications.

According to a further embodiment of the method, the entire body or parts thereof comprise a temperature of at least 20°C, and preferably up to 80°C, more preferably up to 50°C upon mechanically pressing the entire body or parts thereof.

According to a further embodiment, the entire body or parts thereof are mechanically pressed with a pressure of at least 1 N/mm² and preferably up to 20 N/mm² and more preferably up to 10 N/mm².

Furthermore, according to an embodiment, the entire body or parts thereof are mechanically pressed for at least 15 minutes.

According to yet another embodiment of the method according to the present invention, step (iii) of freezing the body comprises subjecting the body for at least six hours to a temperature equal to or below -20°C at atmospheric pressure (i.e. 1013.25 mbar ± 50 mbar).

Further, according to an embodiment, step (iii) of freezing the body comprises subjecting the body for at least six hours to a temperature equal to -45°C or below -45°C at atmospheric pressure (i.e. 1013.25 mbar ± 50 mbar). Further, in an embodiment, step (iv) of freeze drying the body comprises subjecting the body to a pressure of 0.07 mbar or less for at least 48 hours while gradually increasing the temperature to room temperature.

A freezing of the bacterial cellulose over several hours before starting the freeze drying (sublimation of water at reduced pressure), allows for obtaining a porous bacterial cellulose having a higher swelling capacity than without a prior freezing step.

According to a further embodiment of the method according to the present invention, cleaning of the body in step (ii) comprises contacting the body with an alkaline solution, particularly a sodium hydroxide solution. Furthermore, preferably, cleaning of the body in step (ii) comprises rinsing the body with water, particularly multiple times.

Furthermore, in an embodiment of the method, particularly after cleaning of the body in step (ii), the body is cut to size prior to freezing the body in step iii), wherein particularly the body is cut by means of a laser or by means of at least one blade.

Further, in an embodiment, after mechanically pressing the body in step (iv), the body is subjected to a final cut, particularly by means of a laser or at least one blade.

Particularly, according to an embodiment, the body is formed (e.g. with help of cutting the body, see e.g. above) into a patch, a strip, or into a ring.

A further aspect of the present invention relates to a swellable body produced with the method according to the present invention.

According to a further aspect, the present invention relates to a swellable body consisting of bacterial cellulose, the body comprising a swelling factor of at least 1000%, particularly at least 1500%, particularly at least 1600%, particularly at least 1700%, particularly at least 1800%, particularly at least 1900%, particularly at least 2000%, particularly at least 2100%, particularly at least 2200%. The swelling factor (in %) is defined by the ration between the thickness after swelling to the thickness before swelling (x100).

According to yet another aspect of the present invention, the invention relates to a medical implant comprising a swellable body according to one of the aspects of the present invention.

According to an embodiment of the medical implant the swellable body forms a seal of the medical implant (e.g. against paravalvular leakages in a TAVI prosthesis).

According to an embodiment of the medical implant, the swellable body forms at least a portion of a member configured to occlude a cavity (of a patient's anatomy/tissue) such as an atrial auricle.

According to a further embodiment of the medical implant, the medical implant comprises a scaffold (such as a stent), wherein the body is fixed to the scaffold (particularly to an outside of the scaffold/stent). Particularly, the body can form an outer skirt of the scaffold or stent, or a portion of such an outer skirt.

The novel material produced with help of the present invention is advantageous in that it allows new technical solutions for the following classes of implants: (1) implants with integrated sealing of potential leaks by attached, highly swellable material layers; (2) implants for filling cavities, consisting of a support structure and a highly swellable, space-demanding material; (3) implants for closing holes or puncture sites, consisting of a plastically deformable support structure and a highly swellable, space-demanding material.

In the following, further features, advantages and embodiments of the present invention are explained with reference to the Figures, wherein
- Fig. 1: shows a flow diagram of an embodiment of the method according to the present invention,
- Fig. 2: shows photos of square sample patches (20 mm x 20 mm) of a swellable body made from bacterial cellulose visualizing the described behavior of the material according to the invention,
- Fig. 3: shows photos of ring samples visualizing the described behavior of the material according to the invention,
- Fig. 4: shows such a medical implant in form of a TAVI prosthesis comprising a scaffold and a swellable body in form of an outer skirt (dashed) of the prosthesis, the body being produced with the method according to the present invention, and
- Fig. 5: shows a cross-section of a plastically deformable cylindrical support structure (e.g. scaffold or stent) with a swellable body comprising one layer (top left) or two layers (top right) of swellable bacterial cellulose locally attached in a sector of the support structure. By swelling (bottom row), a cavity can be filled in this sector due to the extreme increase in thickness. The volume requirement can be expanded as desired by adding two or more layers of the material (bottom right).

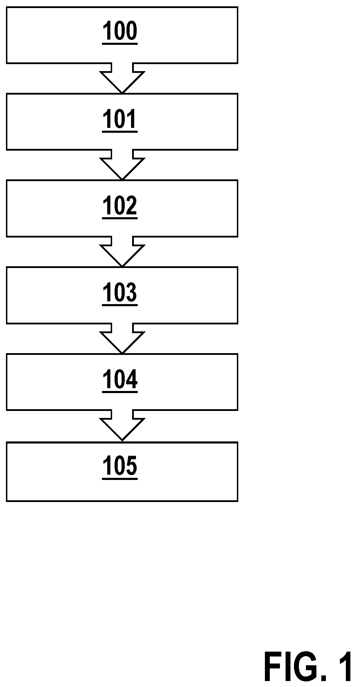

The present invention relates to a method for producing a swellable body 10 comprising bacterial cellulose, wherein the method comprises the steps of (cf. Fig. 1): providing a body 10 made of bacterial cellulose 100, cleaning the body 10 using at least one liquid medium 101, and freeze-drying the body 103.

Particularly, in step 100, according to an embodiment, bacteria from the class acetobacter xylinum can be used to synthesize the bacterial cellulose, particularly nanocellulose, being used in the method according to the present invention, in a nutrient medium under suitable growth conditions (e.g. standard nutrient medium comprising acetobacter, 7 days, 28°C, 90% relative humidity). Particularly, the growth of bacterial nanocellulose in the nutrient medium does not take place in the entire volume but always only at the interface with atmospheric oxygen.

According to an example, the bacterial cellulose is generated using a nutrient medium for acetobacter xylinum having the following composition: 20 g/l glucose, 5 g/l peptone, 5 g/l yeast extract, 2.7 g/l di sodium hydrogen phosphate, 1.5 g citric acid. This culture medium is inoculated with bacteria from the class acetobacter xylinum. In this nutrient medium, bacterial nanocellulose is formed at typically 26°C to 30°C in an incubator over a period of 6 days to 8 days. After 7 days of culture in a dish, a native starting material of about 7 mm to 8 mm thickness is obtained under the above conditions.

The native bacterial cellulose, particularly nanocellulose, can contain large amounts of culture medium as well as the remains of Gram-negative bacteria, which are endotoxins. For this reason, the material is preferably purified in step 101 for typically three days at 80°C in e.g. 0.1 molar sodium hydroxide solution and a large number of rinsing steps in water. The obtained native patch material 10 can be cut into any shape in step 102 before further processing using a scalpel or a CO₂ laser.

The standard "bacterial nanocellulose" material processed in this way has a very high water content being greater than 98%. Due to the absence of cellulases in the human organism, it is not enzymatically or otherwise degradable as an implant material. In this form, it is already approved for clinical applications.

Crucial for the solution according to the invention is the removal of water by means of freezing and freeze drying, since only this process preserves the structure of the fiber network of the cellulose fibrils, i.e. no irreversible structural change occurs as a result of the formation of new intermolecular hydrogen bonds, so-called cornification. The reason for this is that the water contained in the bacterial cellulose is initially frozen before the process of freeze drying, and the removal of the water takes place by sublimation of the solid water. The absence of capillary forces prevents structural changes.

According to an embodiment, freezing in step 103 comprises freezing the material/body 10 for six hours at least -20°C, and 48 hours of drying by means of lyophilization. Particularly, according to an embodiment, a typical freezing process in step 103 involves freezing the native material at least -20°C in a freezer for a sufficiently long period, typically at least six hours. This is followed by transfer to a freeze-drying facility with further freezing for at least six hours at -45°C under atmospheric pressure and drying at e.g. 0,07 mbar for at least 48 hours while gradually increasing the temperature to room temperature. A suitable rate for increasing the temperature is by about 1°C/h. The material 10 obtained is spongy, dimensionally stable and absolutely dry.

The material dried in this way can now be significantly reduced in thickness by pressing or rolling in step 104. Hot pressing at 50°C, 10 N/mm² for 15 minutes typically reduces the thickness by a factor greater than 30. The body/material 10 obtained has a very homogeneous thickness distribution and is mechanically very stable, but still flexible. This allows it to be attached to support structures 2 (e.g. scaffolds, stents etc.) of virtually any shape.

Regarding the above described examples, the pressing parameters can have an influence on the swelling of the freeze-dried body 10 made from bacterial cellulose in that a higher pressing force reduces the swelling capacity, which advantageously also allows the body/material 10 to be adapted to the specific application.

Table 1 shows the values for the swelling behavior of bacterial nanocellulose dried differently before pressing. These samples were cut out from a patch 10 by CO₂ laser and pressed parallel to the growth direction after drying. It can be clearly seen, that surprisingly only the freeze-drying leads to such a strong swelling factor above 22 (i.e. above 2200%).

**Table 1: Measured thicknesses and calculated swelling behavior of bacterial nanocellulose dried differently before pressing (7 days standard culture as patch).**

| Patch material | Freeze drying | Air drying at room temperature | Oven 100°C |
|---|---|---|---|
| Native | 8.50 ± 0.21 mm | 7.98 ± 0.25 mm | 7.78 ± 0.25 mm |
| Dried | 7.41 ± 0.34 mm | 0.16 ± 0.06 mm | 0.16 ± 0.07 mm |
| Pressed | 0.22 ± 0.02 mm | 0.08 ±0.01 mm | 0.12 ± 0.02 mm |
| Rehydrated | 4.89 ± 0.26 mm | 0.18 ± 0.01 mm | 0.14 ± 0.01 mm |
| Swelling factor | 22.23 | 2.25 | 1.16 |

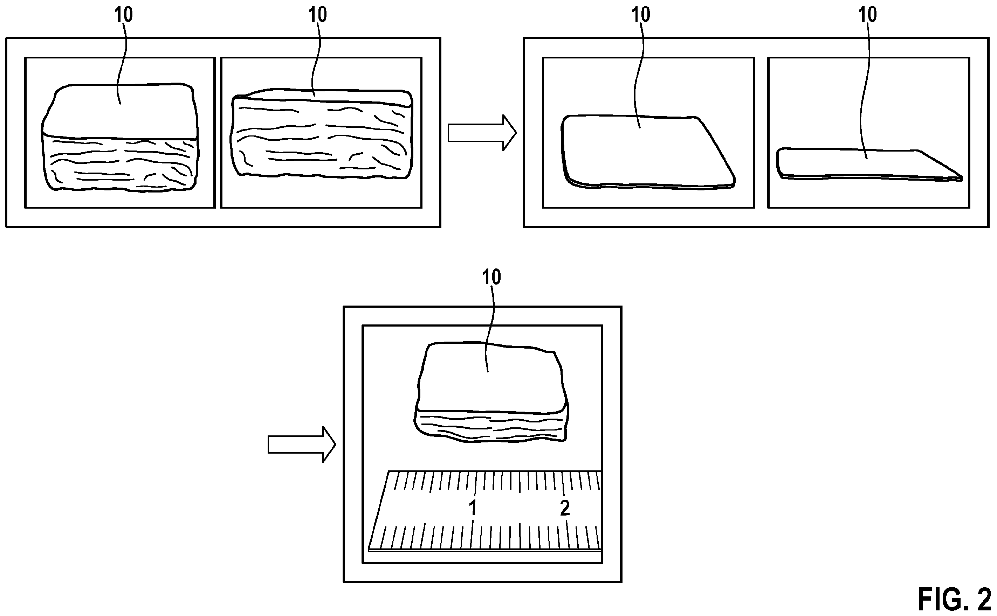

Fig. 2 shows bodies 10 in form of patches of bacterial nanocellulose after drying (48 hours freeze-drying) shown on the left-hand side in the upper row of Fig. 2; after 15 minutes pressing at 10 N/mm² and 50°C (shown on the right-hand side in the upper row of Fig. 2); and after 24 hours rehydration in water (lower row of Fig. 2). The extreme swelling behavior of the material 10 is also present when pressed perpendicular to the growth direction. For this purpose, rings 10 with 5.0 mm and 2.5 mm wall thickness were cut out from the same native patch material, these were then freeze-dried at the same parameters and then manually rolled thin with a cylindrical rod (5 mm diameter). Table 2 shows the values for the swelling behavior. A swelling factor greater than 1000% is also observed for these specimens.

**Table 2: Measured thicknesses and calculated swelling factor of rings 10 of bacterial nanocellulose of different thickness (7 days standard culture as patch; rings cut out with CO₂ laser).**

| | | |
|---|---|---|
| Ring material | 5.0 mm wall thickness | 2.5 mm wall thickness |
| Native | 5.00 mm | 2.50 mm |
| Dried | 3.88 ± 0.01 mm | 1.48 ± 0.02 mm |
| Pressed | 0.38 ± 0.01 mm | 0.18 ± 0.01 mm |
| Rehydrated | 3.98 ± 0.02 mm | 1.85 ± 0.02 mm |
| Swelling factor | 10.47 | 10.28 |

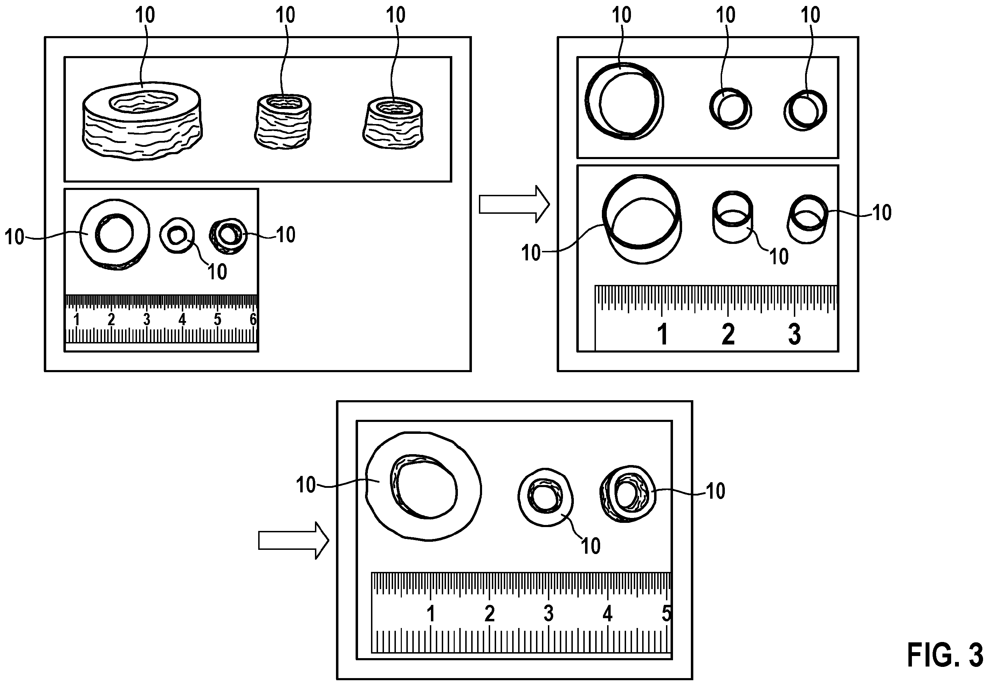

Furthermore, Fig. 3 shows bodies 10 in form of rings of bacterial nanocellulose after 48 hours of freeze drying (left-hand side in the upper row of Fig. 3), after pressing by manual rolling (right-hand side in the upper row of Fig. 3) and after 1 hour of rehydration in water (lower row of Fig. 3).

Swellable bodies 10 according to the invention produced e.g. using the method shown in Fig. 1 can be cut into any shape by final cutting (step 105). For example, rectangular strips can be obtained which, when dry, can be attached to an outside of a stent of a TAVI (transcatheter aortic valve implantation) prosthesis as an outer skirt by conventional suturing. Since the overall system must be dry, this is possible for TAVI prosthesis that use, for example, stabilized dried porcine pericardium as biological material.

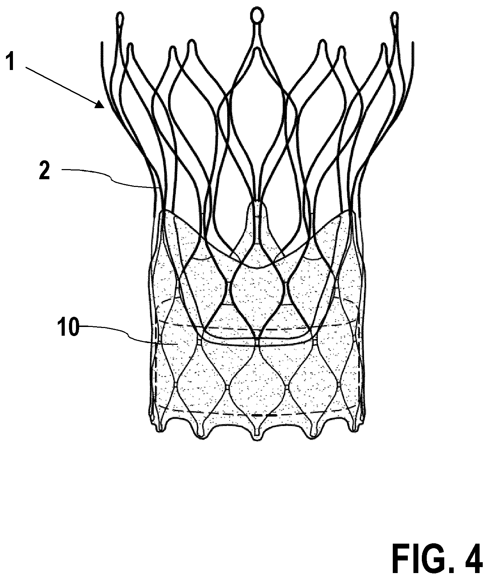

Fig. 4 shows such a TAVI prosthesis 1 comprising a stent 2 comprising interconnected struts forming a circumferential cell structure, wherein an outer skirt 10 is fixed to an outside of the stent 2 that corresponds to a patch or annular body formed out of bacterial cellulose being freeze dried according to the present invention.

The application of molded parts made of the material 10 according to the present invention can be directly transferred to other applications. Optionally, the material/swellable body 10 can also be applied to the inner side of the supporting framework 2. Likewise, only parts of the implant surface can be covered with the swellable material/body 10.

Particularly, a swellable body 10 made from bacterial cellulose according to the invention can also be used in an implant for filling cavities. This is due to the fact that the swelling factor of the respective swellable body 10 according to the present invention is extremely high, and a 0.2 mm thick dry body 10 results in a body 10 about 5 mm thick in the swollen state. This can be extended as required by providing a body 10 comprising several separate layers 10a, 10b of the freeze-dried bacterial cellulose 10. Therefore, the material/body 10 according to the present invention also allows to fill large volumes by minimally invasive, catheter-based implantation of a stent 2 with a swellable body/material 10 appropriately attached to an outside of the stent 2. Stabilization of aneurysms with such a system 1 is conceivable. Advantageously, this principle is also transferable to other applications. Thus, an improvement of implants for the closure of the cardiac ear in the left atrium is possible by using the body/material 10 according to the invention.

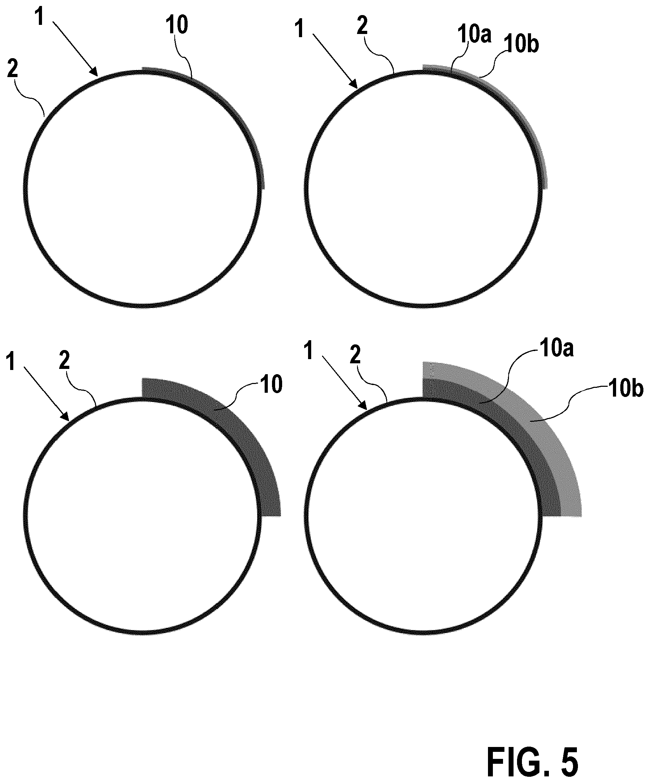

Furthermore, Fig. 5 shows a cross-sectional view of a plastically deformable cylindrical support structure (e.g. scaffold or stent) comprising a body 10 with one layer (top left) or two layers 10a, 10b (top right) of extremely swellable bacterial cellulose 10 formed according to the method according to the present invention and being locally attached to a sector of the support structure 2 on an outside of the structure 2. By swelling (bottom row), a cavity can be filled in the respective sector due to the extreme increase in thickness of the body 10 attached to the support structure 2. The volume requirement can be expanded as desired by adding two or more layers 10a, 10b of the material (bottom right).

By combining the material/body 10 according to the invention with a deformable retaining structure, a significant improvement of implants for closing holes is conceivable. Examples include closure systems for punctures with a plastically deformable clip structure and closure systems for ventricular septal defects with a plastically super-elastic holding structure made of Nitinol. Due to the extremely strong swelling of the material/body 10, a more secure and stable closure of holes is possible than with non-swellable tissues. The bacterial nanocellulose is also stable over the long term and provides a natural barrier to microorganisms.

In a very general way, the method according to the present invention allows the production of an exceptionally swellable biomaterial that can assume virtually any three-dimensional form in the swollen state. With such a material, enormous technical advantages arise for the realization of implants that (1) incorporate such a body 10 as an integrated seal for leakage or a part thereof, or (2) as an element being capable of filling cavities, or (3) as an element being configured to selectively close openings.

The method according to the present invention can be applied to all types of native bacterial cellulose, i.e., regardless of bacterial strain and cultivation conditions.

Furthermore, advantageously, implementation of the present invention in an approved medical manufacturing process is very straightforward, as established techniques can be used and no other substances have to be introduced into the pure bacterial nanocellulose. The swelling capacity of the material can be adapted to the requirements of the respective medical implant by means of the pressing parameters (e.g. pressing pressure, temperature of the body 10 upon pressing, duration of the pressing). Molded parts made from the material 10 produced according to the present invention can be very easily attached to retaining structures 2 using existing methods. Furthermore, bacterial nanocellulose has potential advantages over xenogeneic materials commonly used in biological implants, such as porcine pericardium, in terms of tendency to calcify, homogeneity of material properties, reduced thickness with comparable mechanical properties, production in virtually any shape, stability to biodegradation without additional chemical fixation.

## Claims

1. A method for producing a swellable body (10) comprising bacterial cellulose, wherein the method comprises the steps of:
(i) providing a body (10) made of bacterial cellulose,
(ii) cleaning the body (10) using at least one liquid medium, and
(iii) freezing the body at atmospheric pressure for at least six hours, and
(iv) freeze drying the body (10), and
(v) mechanically pressing the entire body (10) or parts thereof after freeze drying of the body (10).

2. The method according to claim 1, wherein the entire body (10) or parts thereof comprise a temperature of at least 20°C upon mechanically pressing the entire body (10) or parts thereof, and/or wherein the entire body (10) or parts thereof are mechanically pressed with a pressure of at least 1 N/mm², and/or wherein the entire body (10) or parts thereof are mechanically pressed for at least 15 minutes.

3. The method according to one of the preceding claims, wherein step (iii) of freezing the body (10) comprises subjecting the body (10) for at least six hours to a temperature equal to or below -20°C at atmospheric pressure, and/or wherein step (iv) of freeze drying the body (10) comprises subjecting the body (10) to a pressure of 0.07 mbar or less for at least 48 hours while increasing the temperature of the body (10) to room temperature

4. The method according to one of the preceding claims, wherein step (iii) of freezing the body (10) comprises subjecting the body (10) for at least six hours to a temperature equal to or below -45°C at atmospheric pressure, and/or wherein step (iv) of freeze drying the body (10) comprises subjecting the body (10) to a pressure of 0.07 mbar or less for at least 48 hours while increasing the temperature of the body (10) to room temperature.

5. The method according to one of the preceding claims, wherein cleaning the body (10) in step (ii) comprises contacting the body (10) with an alkaline solution, particularly a sodium hydroxide solution, and/or wherein cleaning the body (10) in step (ii) comprises rinsing the body (10) with water.

6. The method according to one of the claims 1 to 5, wherein prior to freezing the body (10) in step (iii), the body (10) is cut to size, particularly by means of a laser or a blade.

7. The method according to one of the claims 1 to 3, wherein after mechanically pressing the body (10) in step (iv), the body (10) is subjected to a final cut, particularly by means of a laser or a blade.

8. The method according to one of the preceding claims, wherein the body (10) is formed into one of: a patch, a strip, a ring.

9. A swellable body (10) produced with the method according to one of the claims 1 to 8.

10. A medical implant (1) comprising the swellable body (10) according to claim 9.

11. The medical implant (1) according to claim 10, wherein the swellable body (10) forms a seal of the medical implant (1).

12. The medical implant according to claim 10, wherein the swellable body (10) forms at least a portion of a member configured to occlude a cavity.

13. The medical implant according to one of the claims 10 to 12, wherein the medical implant (1) comprises a scaffold (2), wherein the swellable body (10) is fixed to the scaffold (2).

## Patentansprüche

1. Verfahren zur Herstellung eines quellfähigen Körpers (10), der bakterielle Cellulose umfasst, wobei das Verfahren die folgenden Schritte umfasst:
(i) Bereitstellung eines Körpers (10) aus bakterieller Zellulose,
(ii) Reinigen des Körpers (10) mit mindestens einem flüssigen Medium, und
(iii) Einfrieren des Körpers bei Atmosphärendruck für mindestens sechs Stunden, und
(iv) Gefriertrocknung des Körpers (10), und
(v) mechanisches Pressen des gesamten Körpers (10) oder von Teilen davon nach der Gefriertrocknung des Körpers (10).

2. Verfahren nach Anspruch 1, wobei der gesamte Körper (10) oder Teile davon beim mechanischen Pressen des gesamten Körpers (10) oder von Teilen davon eine Temperatur von mindestens 20° C aufweisen, und/oder wobei der gesamte Körper (10) oder Teile davon mit einem Druck von mindestens 1 N/mm² mechanisch gepresst werden, und/oder wobei der gesamte Körper (10) oder Teile davon mindestens 15 Minuten lang mechanisch gepresst werden.

3. Verfahren nach einem der vorhergehenden Ansprüche, wobei Schritt (iii) des Einfrierens des Körpers (10) das Aussetzen des Körpers (10) für mindestens sechs Stunden einer Temperatur von -20°C oder weniger bei Atmosphärendruck umfasst, und/oder wobei Schritt (iv) des Gefriertrocknens des Körpers (10) das Aussetzen des Körpers (10) einem Druck von 0,07 mbar oder weniger für mindestens 48 Stunden umfasst, während die Temperatur des Körpers (10) auf Raumtemperatur erhöht wird.

4. Verfahren nach einem der vorhergehenden Ansprüche, wobei Schritt (iii) des Einfrierens des Körpers (10) das Aussetzen des Körpers (10) für mindestens sechs Stunden einer Temperatur gleich oder unter -45°C bei atmosphärischem Druck umfasst, und/oder wobei Schritt (iv) des Gefriertrocknens des Körpers (10) das Aussetzen des Körpers (10) einem Druck von 0,07 mbar oder weniger für mindestens 48 Stunden umfasst, während die Temperatur des Körpers (10) auf Raumtemperatur erhöht wird.

5. Verfahren nach einem der vorhergehenden Ansprüche, wobei die Reinigung des Körpers (10) in Schritt (ii) das Inkontaktbringen des Körpers (10) mit einer alkalischen Lösung, insbesondere einer Natriumhydroxidlösung, umfasst, und/oder wobei die Reinigung des Körpers (10) in Schritt (ii) das Spülen des Körpers (10) mit Wasser umfasst.

6. Verfahren nach einem der Ansprüche 1 bis 5, wobei vor dem Einfrieren des Körpers (10) in Schritt (iii) der Körper (10) zugeschnitten wird, insbesondere mittels eines Lasers oder einer Klinge.

7. Verfahren nach einem der Ansprüche 1 bis 3, wobei nach dem mechanischen Pressen des Körpers (10) in Schritt (iv) der Körper (10) einem Endschnitt, insbesondere mittels eines Lasers oder einer Klinge, unterzogen wird.

8. Verfahren nach einem der vorhergehenden Ansprüche, wobei der Körper (10) zu einem der folgenden Elemente geformt wird: einem Pflaster, einem Streifen, einem Ring.

9. Quellbarer Körper (10), hergestellt nach dem Verfahren gemäß einem der Ansprüche 1 bis 8.

10. Medizinisches Implantat (1) mit dem quellfähigen Körper (10) nach Anspruch 9.

11. Medizinisches Implantat (1) nach Anspruch 10, wobei der quellfähige Körper (10) eine Abdichtung des medizinischen Implantats (1) bildet.

12. Medizinisches Implantat nach Anspruch 10, wobei der quellfähige Körper (10) mindestens einen Teil eines Elements bildet, das zum Verschließen eines Hohlraums konfiguriert ist.

13. Medizinisches Implantat nach einem der Ansprüche 10 bis 12, wobei das medizinische Implantat (1) ein Gerüst (2) umfasst, wobei der quellfähige Körper (10) an dem Gerüst (2) befestigt ist.

## Revendications

1. Procédé de production d'un corps dilatable (10) comprenant de la cellulose bactérienne, dans lequel le procédé comprend les étapes consistant à :
(i) fournir un corps (10) fabriqué en cellulose bactérienne,
(ii) nettoyer le corps (10) en utilisant au moins un milieu liquide, et
(iii) congeler le corps à pression atmosphérique pendant au moins six heures, et
(iv) lyophiliser le corps (10), et
(v) presser mécaniquement le corps (10) entier ou des parties de celui-ci après avoir lyophilisé le corps (10).

2. Procédé selon la revendication 1, dans lequel le corps (10) entier ou des parties de celui-ci comprennent une température d'au moins 20 °C lors de l'étape consistant à presser mécaniquement le corps (10) entier ou des parties de celui-ci, et/ou dans lequel le corps (10) entier ou des parties de celui-ci sont pressés mécaniquement avec une pression d'au moins 1 N/mm², et/ou dans lequel le corps (10) entier ou des parties de celui-ci sont pressés mécaniquement pendant au moins 15 minutes.

3. Procédé selon l'une des revendications précédentes, dans lequel l'étape (iii) consistant à congeler le corps (10) comprend une étape consistant à soumettre le corps (10) pendant au moins six heures à une température inférieure ou égale à -20 °C à pression atmosphérique, et/ou dans lequel l'étape (iv) consistant à lyophiliser le corps (10) comprend une étape consistant à soumettre le corps (10) à une pression de 0,07 mbar ou moins pendant au moins 48 heures tout en augmentant la température du corps (10) jusqu'à la température ambiante.

4. Procédé selon l'une des revendications précédentes, dans lequel l'étape (iii) consistant à congeler le corps (10) comprend une étape consistant à soumettre le corps (10) pendant au moins six heures à une température inférieure ou égale à -45 °C à pression atmosphérique, et/ou dans lequel l'étape (iv) consistant à lyophiliser le corps (10) comprend une étape consistant à soumettre le corps (10) à une pression de 0,07 mbar ou moins pendant au moins 48 heures tout en augmentant la température du corps (10) jusqu'à la température ambiante.

5. Procédé selon l'une des revendications précédentes, dans lequel l'étape consistant à nettoyer le corps (10) dans l'étape (ii) comprend une étape consistant à mettre en contacte le corps (10) avec une solution basique, en particulier une solution d'hydroxyde de sodium, et/ou dans lequel le nettoyage du corps (10) dans l'étape (ii) comprend une étape consistant à rincer le corps (10) avec de l'eau.

6. Procédé selon l'une des revendications 1 à 5, dans lequel avant l'étape consistant à congeler le corps (10) dans l'étape (iii), le corps (10) est coupé à taille, en particulier au moyen d'un laser ou d'une lame.

7. Procédé selon l'une des revendications 1 à 3, dans lequel après l'étape consistant à presser mécaniquement le corps (10) dans l'étape (iv), le corps (10) est soumis à une coupe finale, en particulier au moyen d'un laser ou d'une lame.

8. Procédé selon l'une des revendications précédentes, dans lequel le corps (10) est formé en l'un parmi : un timbre, une bande, un anneau.

9. Corps dilatable (10) produit avec le procédé selon l'une des revendications 1 à 8.

10. Implant médical (1) comprenant le corps dilatable (10) selon la revendication 9.

11. Implant médical (1) selon la revendication 10, dans lequel le corps dilatable (10) forme un joint de l'implant médical (1).

12. Implant médical selon la revendication 10, dans lequel le corps dilatable (10) forme au moins une partie d'un élément conçu pour boucher une cavité.

13. Implant médical selon l'une des revendications 10 à 12, dans lequel l'implant médical (1) comprend un échafaudage (2), dans lequel le corps dilatable (10) est fixé à l'échafaudage (2).
